# EUROPÄISCHE PATENTANMELDUNG

(11) **EP 4 026 889 A1**
(43) Veröffentlichungstag der Anmeldung: **13.07.2022**
(21) Anmeldenummer: 21150942.7
(22) Anmeldetag: 11.01.2021
(51) Int. Cl.: C12M 1/00, C12M 1/26, C12M 1/12

(54) **STERILSCHUTZ-AUFSATZ FÜR SPITZEN VON LIQUID-HANDLING-EINRICHTUNGEN**

(71) Anmelder: Eppendorf AG, 22339 Hamburg (DE)
(72) Erfinder: Rix, Karl, 42555 Velbert (DE); Selzer, Sebastian, 3400 Klosterneuburg (AT); Schneider, Falk, 52072 Aachen (DE); Schüler, Kai, CH-9114 Hoffeld SG (CH); Schillig, Christophe, CH-8625 Gossau ZH (CH)
(74) Vertreter: Eisenführ Speiser

(57) **Zusammenfassung**

Die Erfindung betrifft einen Sterilschutz-Aufsatz für eine Spitze einer Liquid-Handling-Einrichtung umfassend eine Halterung zur Befestigung des Sterilschutz-Aufsatzes an der Liquid-Handling-Einrichtung, eine in der Halterung angeordnete Schutzglocke mit einem inneren Hohlraum sowie eine mit dem Hohlraum verbundene Zuleitung für sterile Luft, bei dem die Schutzglocke an einem unteren Ende eine Öffnung aufweist und an einer dem unteren Ende gegenüberliegenden oberen Seite eine Aufnahme für die Spitze aufweist.

## Beschreibung

Die Erfindung betrifft einen Sterilschutz-Aufsatz für eine Spitze einer Liquid-Handling-Einrichtung, eine Sterilschutzeinheit, ein Liquid-Handling-System, ein Verfahren zum Betrieb eines Sterilschutz-Aufsatzes sowie ein Verfahren zum Befüllen oder Entnehmen mit einer Sterilschutzeinheit und mit einem Liquid-Handling-System.

Im Markt sind typischerweise Autosampler oder auch Pipettierroboter anzutreffen, die den Vorgang der Probenaufbereitung und Verteilung an eine nachgelagerte meist sequentiell arbeitende Analytik automatisieren. Die Proben stehen dabei meist bereits in geeigneten Kleinstgefäßen zur Verfügung. Bei Bioprozessen müssen häufig Proben mehrfach während der Kultivierung im Bioreaktor aus diesem möglichst steril entnommen werden und zwischengelagert oder einer Analytik direkt (online) zugeführt werden. Da zunehmend mehrere Bioreaktoren parallel betrieben werden, soll die Probenahme aus mehreren Reaktoren über ein Probenahmesystem (ausgeführt oft als Multiplexer) erfolgen. Hierbei sind meist erhöhte Sterilanforderungen, insbesondere bei der Entnahme aus dem Bioreaktor zu erfüllen. Die Möglichkeit einer Kontamination aus der Umgebung oder zwischen unterschiedlichen Bioreaktoren (Kreuzkontamination) muss sicher verhindert werden können. Online-Probenahmesysteme können als schlauchgeführte geschlossene Systeme mit Pumpen und Ventilen oder Liquid-Handling-Systeme mit Mehrweg-Spritzen aus Glas oder Einwegspitzen aus Kunststoff ausgeführt werden. In geschlossenen Systemen ist oft die Kontamination bzw. Reinigung der Schläuche oder das Totvolumen problematisch. Liquid-Handling-Systeme können zudem auch verwendet werden um mehreren Bioreaktoren gezielt (z. B. basierend auf einem ausgewerteten, analytischen Ergebnis einer zuvor entnommenen Probe) während der Kultivierung Substanzen zuzugeben. Auch in diesem Fall ist eine sicherere Verhinderung von Kontaminationen zwingend erforderlich.

Bekannte Liquid-Handling-Systeme müssen oft in einer sterilen Einhausung mit dem Bioreaktor zusammen betrieben werden, um eine Kontamination sicher zu verhindern. Dies ist aufgrund des Aufwands, der räumlichen Ausdehnung sowie der Einschränkungen im Handling durch das Laborpersonal meist unerwünscht.

Hier setzt die Erfindung an, deren Aufgabe es ist, unabhängig von der Umgebung ein steriles Arbeiten, insbesondere mit Liquid-Handling-Systemen, zu ermöglichen und die genannten Probleme zu adressieren.

Gemäß einem ersten Aspekt betrifft die Erfindung einen Sterilschutz-Aufsatz für eine Spitze einer Liquid-Handling-Einrichtung umfassend eine Halterung zur Befestigung des Sterilschutz-Aufsatzes an der Liquid-Handling-Einrichtung, eine in der Halterung angeordnete Schutzglocke mit einem inneren Hohlraum sowie eine mit dem Hohlraum verbundene Zuleitung für sterile Luft. Die Schutzglocke weist dabei an einem unteren Ende eine Öffnung auf und an einer dem unteren Ende gegenüberliegenden oberen Seite eine Aufnahme für die Spitze.

Der Erfindung liegt die Erkenntnis zugrunde, dass ein steriles Arbeiten mit einer Spitze auch in nicht-sterilen Umgebungen möglich wird, wenn die Spitze dauerhaft durch einen Sterilschutz-Aufsatz geschützt wird. Hierzu wird eine Schutzglocke an der Spitze angeordnet, innerhalb derer die Spitze dauerhaft mit steriler Luft gespült werden kann. Es wird innerhalb der Schutzglocke also ein steriler Luftvorhang etabliert. Somit wird die Spitze gegen die Umgebung gekapselt und kann auch in nicht-steriler Umgebung bewegt und verwendet werden, ohne beim Eindringen in ein Gefäß dieses zu kontaminieren.

Die Erfindung ermöglicht also automatisiertes, steriles Arbeiten mit Spitzen in nicht-sterilen Umgebungen bei der Befüllung mit oder der Entnahme von Materialien beispielsweise in Bioreaktoren.

Unter Spitzen werden neben spitzen Hohlnadeln auch stumpfere Spitzen wie Pipettenspitzen oder männliche Verbindungsstücke verstanden. Penetrieren meint im Rahmen dieser Erfindung neben Durchstechen auch das Aufdrücken selbstschließender Membranen, wie beispielsweise in swabable valves, auch als abwischbare Ventile oder nadelfreie Membranventile bezeichnet, durch stumpfe Spitzen, beispielsweise männliche Verbindungsstücke. Unter einer Liquid-Handling-Einrichtung werden sämtliche Arten von Liquid-Handling-Einrichtungen verstanden, insbesondere auch Einzelpipetten, Einzelspritzen, Einzelleitungen mit Spitze, Handlingroboter, aber auch (automatisierte) Mehrfachsysteme.

In einer Ausführungsform des Sterilschutz-Aufsatzes ist die Aufnahme eine Öffnung, durch die die Spitze geführt werden kann. Die Spülung des inneren Hohlraumes mit steriler Luft verhindert das Eindringen von nicht-steriler Umgebungsluft in den inneren Hohlraum und die Spitze bleibt somit geschützt. Es ist hier insbesondere vorteilhaft, wenn über die Zuleitung ein stetiger Luftstrom in den Hohlraum eingebracht wird, der einen geringen Überdruck im Vergleich zur Umgebung im Hohlraum erzeugt, beispielsweise ist es vorteilhaft, wenn bei 1 bar Umgebungsdruck der Druck innerhalb des Hohlraums zwischen 1 und 1,5 bar liegt. Insbesondere durch den geringen Überdruck wird somit das Einströmen unsteriler Luft von außen sicher verhindert und gleichzeitig die Spitze dauerhaft umspült.

Alternativ ist die Aufnahme ausgebildet, die Spitze gas- und flüssigkeitsdicht zu umschließen und verhindert so ein Eindringen der Umgebungsluft in den inneren Hohlraum an der Stelle der Aufnahme.

In einer Weiterbildung ist die Aufnahme dabei als eine selbstschließende Membran ausgebildet. Die selbstschließende Membran ist in einer Ausführungsform als durchstechbares Septum ausgebildet, also als ein Septum, dass auch bei Penetration außerhalb der Penetration undurchlässig ist und mit dem eindringenden Gegenstand, also der Spitze einen dichten Abschluss bildet. Es ist insbesondere bevorzugt, wenn das durchstechbare Septum für Gase und Flüssigkeiten undurchlässig ist, auch wenn das Septum von der Spitze, insbesondere einer Hohlnadel oder einer Pipettenspitze, insbesondere von einer Hohlnadel oder Pipettenspitze mit einem Durchmesser unter 1,5 mm, penetriert wird und auch nach mehrfacher Penetration durch eine derartige Hohlnadel oder Pipettenspitze. Das durchstechbare Septum weist in einer Ausführungsform einen Schlitz, insbesondere einen Kreuzschlitz für die Penetration auf. Das Septum kann beispielsweise aus Silikon, Naturkautschuk, PTFE, TPE oder anderen Elastomeren gefertigt sein, derartige Septen sind aus dem Stand der Technik bereits bekannt. Alternativ kann die Aufnahme eine Dichtung aufweisen, mit der der Innenraum der Schutzglocke gegen den Außenraum an der Stelle der Aufnahme gas- und flüssigkeitsdicht abgeschlossen ist. Mit beiden Ausführungsformen wird gewährleistet, dass keine verunreinigte Luft an der Stelle der Aufnahme unter die Schutzglocke eindringen kann. Dies wird weiter unterstützt von der Spülung mit steriler Luft, die verhindert, dass Umgebungsluft unter die Schutzglocke gelangt.

In einer weiteren Ausführungsform ist die Aufnahme derart elastisch ausgebildet, dass bei einem geringen Verfahrweg der Spitze, insbesondere bei Verfahrwegen von weniger als 2 cm, ein Teil der Aufnahme mit der Spitze bewegt wird. Mit dieser Ausführungsform wird bei kurzen Verfahrwegen beim Penetrieren eines Gefäßes oder Bioreaktors oder des jeweiligen Anschlusses sichergestellt, dass kein Teil der Spitze beim Rausziehen aus dem Gefäß oder Bioreaktor oder der des jeweiligen Anschlusses mit der Umgebungsluft in Kontakt kommt und später wieder in den Innenraum der Schutzglocke gelangt. Damit wird die Sterilwirkung des Sterilschutz-Aufsatzes weiter verbessert, ohne einen Reinigungsschritt zwischen Penetrationen der Spitze in Gefäße oder Anschlüsse vorsehen zu müssen.

Darüber hinaus weist die Aufnahme in einer Ausführungsform eine Dicke auf, die größer ist als der maximale Verfahrweg der Spitze im Betrieb. Auch hierüber wird sichergestellt, dass Teile der Spitze, die die Schutzglocke beim Verfahren verlassen haben nicht wieder in den Innenraum gelangen, sondern nur in die Aufnahme.

In einer weiteren Ausführungsform ist die Schutzglocke an ihrem unteren Ende derart ausgebildet, dass sie bei Aufsetzen auf ein Gefäß oder Anschluss mit diesem dicht abschließt, insbesondere gasdicht abschließt. Zusätzlich zur Spülung des Innenraums der Schutzglocke mit steriler Luft zur Kapselung der Spitze gegen die Umgebungsluft wird bei dieser Ausführungsform beim Aufsetzen über den dichten Abschluss sichergestellt, dass keine Umgebungsluft die Spitze erreicht. Ein dichtes Aufsetzen der Schutzglocke ist nur vorteilhaft, wenn sichergestellt ist, dass das Gefäß oder Anschluss selbst, frei von möglichen Kontaminationen ist. Alternativ ist es vorteilhaft, wenn die Schutzglocke beim Eindringen der Spitze in ein Gefäß oder Anschluss einen vorgegebenen Abstand zum Gefäß einhält, so dass es zu keiner Berührung von Schutzglocke und Gefäß kommt.

Es ist vorteilhaft, wenn die Schutzglocke an ihrem unteren Ende elastisch ausgebildet ist. Darüber kann zum einen ein besserer Abschluss bei einem Aufsetzen auf ein Gefäß oder Anschluss erreicht werden und zum anderen, je nach konkreter Ausformung, ein weiterer Schutz der Spitze im nicht aufgesetzten Zustand, beispielsweise durch einfaches nach innen Bewegen des unteren Endes, erfolgen.

Die Öffnung der Schutzglocke weist dabei beispielsweise einen Kreuzschlitz auf, der sich vorteilhaft auch über eine bestimmte Länge der Spitze erstreckt. Wird die Spitze nun in Richtung eines Gefäßes oder Anschlusses und damit die Schutzglocke auf das Gefäß oder den Anschluss gedrückt, wird auch die Öffnung der Schutzglocke aufgedrückt. Diese lässt dann die Spitze frei das Gefäß penetrieren schließt zusammen mit der sterilen Luft im inneren Hohlraum die Spitze gegen die Umgebungsluft ab. Sobald die Spitze zurückgezogen wird, schließt sich der Kreuzschlitz weitgehend wieder. Alternativ ist beispielsweise auch eine kreisrunde Öffnung möglich.

Die Schutzglocke ist vorteilhaft ganz oder teilweise aus Polyetheretherketon (PEEK) gefertigt. PEEK ist ausreichend mechanisch stabil und elastisch und zusätzlich gut, auch mehrfach autoklavierbar.

Alternativ kann das untere Ende der Schutzglocke formschlüssig zu einem bestimmten Gefäß- oder Anschlusstyp, insbesondere einer bestimmten Art von Bioreaktoren ausgebildet sein, um einen dichten Abschluss zu gewährleisten. Auch hier ist beispielsweise eine kreisrunde Öffnung möglich.

Vorteilhaft ist es weiterhin, wenn die Halterung längenverstellbar, insbesondere in Teleskopform, ausgeführt ist. Damit kann der Sterilschutz-Aufsatz flexibel an verschiedene Liquid-Handling-Einrichtung mit unterschiedlichen Abmessungen adaptiert und mit diesen eingesetzt werden. Hierüber ist insbesondere auch eine Nachrüstung bestehender Liquid-Handling-Einrichtungen möglich

In einer Ausführungsform umfasst die Zuleitung für sterile Luft einen sterilen Filter, insbesondere einen sterilen Filter mit einem Porendurchmesser kleiner oder gleich 0,22 µm, insbesondere einen Spitzenvorsatz-Filter. Alternativ ist die Zuleitung mit einem Reservoir für sterile Luft verbindbar. Die Zuleitung ist weiter vorteilhaft derart ausgebildet, dass eine Umspülung mit steriler Luft dauerhaft erfolgen kann oder zumindest während eines Eindringens der Spitze bis zur vollständigen Penetration in ein Gefäß oder Anschluss und solange die Spitze kein Gefäß und keinen Anschluss penetriert.

Gemäß einem zweiten Aspekt betrifft die Erfindung eine Sterilschutzeinheit umfassend einen Sterilschutz-Aufsatz gemäß dem ersten Aspekt der Erfindung sowie eine in der Aufnahme der Schutzglocke angeordnete Spitze einer Liquid-Handling-Einrichtung.

In einer bevorzugten Ausführungsform der Sterilschutzeinheit ist die Halterung derart ausgebildet und angeordnet, dass ein unteres offenes Ende der Spitze im Betrieb dauerhaft innerhalb des Hohlraums der Schutzglocke angeordnet ist und - nur bei Penetration der Spitze in ein Gefäß oder einen Anschluss- unterhalb des Hohlraums der Schutzglocke angeordnet ist. Darüber wird die Sterilität der Spitze zusätzlich abgesichert. Ein solcher Anschluss eines Gefäßes ist vorteilhaft ein Multi-Connector-Port umfassend einen Luftanschluss für sterile Luft sowie mindestens ein Verbindungsstück, mindestens einen am Verbindungsstück angeordneten Zugang sowie mindestens eine am Verbindungsstück angeordnete Leitung, wobei der Zugang eine selbstschließende Membran umfasst. Mit diesem lässt sich ein steriles Arbeiten in nicht steriler Umgebung besonders gut realisieren.

In einer weiteren Ausführungsform ist die Spitze im Betrieb relativ zur Schutzglocke beweglich angeordnet. Somit kann die Spitze verfahren werden, um einen Anschluss oder ein Gefäß zu penetrieren, ohne dass die Schutzglocke notwendigerweise an ihrem unteren Ende elastisch ist.

Es ist dabei vorteilhaft, wenn die Sterilschutzeinheit einen Anschlag aufweist, der ein Verfahren des Endes der Spitze nach oben aus dem Innenraum der Schutzglocke verhindert. Darüber wird ein Kontakt des Endes der Spitze mit der Umgebungsluft und damit eine Kontamination durch mögliche Fehlbedienungen verhindert. Beispielsweise kann der Anschlag ein mechanischer Anschlag sein oder der Anschlag ausgebildet ist, bei Berührung ein elektronisches Stoppsignal an eine Steuerung der Liquid-Handling-Einrichtung zu senden. Darüber hinaus sind insbesondere bei automatisierten Liquid-Handling-Einrichtungen weitere Absicherungen möglich, wie die Einstellung maximaler Verfahrwege oder dergleichen.

Alternativ ist die Spitze starr zur Halterung angeordnet und das untere Ende der Schutzglocke elastisch ausgebildet ist. Auch hierüber wird eine Penetration ermöglicht bei gleichzeitiger Aufrechterhaltung der Sterilität.

Bevorzugt ist die Sterilschutzeinheit sterilisierbar ausgeführt, wobei die Sterilisation bevorzugt über Autoklavieren, Bestrahlung oder mit Ethylenoxid erfolgen kann. So kann durch einfache Sterilisationsmethoden ein steriler Zugang über die Sterilschutzeinheit realisiert werden, ohne dass Arbeiten in abgeschlossenen Systemen oder Arbeitsstationen notwendig sind.

Gemäß einem dritten Aspekt betrifft die Erfindung ein Liquid-Handling-System umfassend mindestens eine Liquid-Handling-Einrichtung mit mindestens einer Sterilschutzeinheit gemäß dem zweiten Aspekt der Erfindung.

Vorteilhaft umfasst das Liquid-Handling System weiter mindestens einen Multi-Connector-Port, wobei der Multi-Connector-Port einen Luftanschluss für sterile Luft sowie mindestens einen an einem Verbindungsstück angeordneten Zugang, der eine selbstschließende Membran umfasst und mindestens eine am Verbindungstück angeordnete Leitung umfasst.

Insbesondere diesem Aspekt der Erfindung liegt die weitere Erkenntnis zugrunde, dass automatisierte Zugaben und Entnahmen insbesondere in Bioreaktoren und Gefäße, ohne die Notwendigkeit in sterilen Arbeitsstationen oder abgeschlossenen Systemen zu arbeiten und für eine Vielzahl von Ausführungsformen von Bioreaktoren und Gefäßen, möglich sind, wenn die Zugabe- oder Entnahmestelle vom Reaktor oder Gefäß entkoppelt wird. Hierzu wird zwischen Bioreaktor und Liquid-Handling Einrichtung ein Multi-Connector-Port eingesetzt, der mit dem Bioreaktor über mindestens eine Leitung steril und dicht verbunden werden kann und der Zugänge für die Liquid-Handling Einrichtung bereitstellt, die von Spitzen unter Abschluss nach außen penetrierbar sind. Zusammen mit dem Sterilschutz-Aufsatz gemäß dem ersten Aspekt der Erfindung ergibt sich somit ein besonders flexibles System, dass auch in nicht-sterilen Umgebungen sterile Applikationen und/oder Entnahmen mit einer Vielzahl verschiedener Bioreaktoren und Gefäße ermöglicht.

Mit Hilfe des Multi-Connector-Ports können weiter Totvolumina oder Kontaminationen durch vorherige Zu- oder Entnahmevorgänge verhindert oder minimiert werden, wenn der Multi-Connector-Port über einen Anschluss für sterile Luft gespült werden kann, so dass im Verbindungsstück des Multi-Connector-Ports oder in der Leitung verbliebene Flüssigkeit in den Reaktor oder das Gefäß befördert werden kann. Somit kann auch mit sehr kleinen Proben- oder Zugabevolumina gearbeitet werden, da diese vollständig ausgenutzt werden können. Der erfindungsgemäße Multi-Connector-Port und dessen Verwendung stellen also eine Lösung bereit, die flexibel eingesetzt werden kann, keine sterile Umgebung erfordert und Totvolumina vermeidet.

Auch eine Vielzahl von Arbeitsschritten wird mit einem Liquid-Handling-System mit einem Multi-Connector-Port einfacher möglich. So können mit dem Multi-Connector-Port beispielsweise Probenentnahme aus einem Bioreaktor, Probenabgabe in Mikroreaktionsgefäße für nachfolgende Analysen, Probenabgabe in einen Auffangbehälter, Probenabgabe in ein automatisiertes System, wie beispielsweise ein Analysegerät, Ausbluten eines Bioreaktors (Entfernung von Zellen aus dem Bioreaktor, um die Zellkonzentration zu steuern), Zugabe eines Mediums oder eines Medien-Cocktails beispielsweise in einen Bioreaktor realisiert werden. Des Weiteren ist es möglich Medien oder Medien-Cocktails für in den Bioprozess automatisiert und zeitgesteuert zuzuführen.

Der Luftanschluss kann dabei entweder so ausgeführt sein, dass über ihn sterile Luft in die Spitze gezogen werden kann und dann von der Spitze in den mindestens einen Zugang abgegeben wird, der Luftanschluss ist dann separat und fluidisch nicht mit dem mindestens einen Zugang verbunden oder der Luftanschluss ist direkt fluidisch mit dem mindestens einen Verbindungsstück und damit mit dem mindestens einen Zugang verbunden.

In einer Ausführungsform hat daher eine der selbstschließenden Membran abgewandte Seite des sterilen Filters Kontakt zur Umgebungsluft. Wird in dieser Ausführungsform eine Spitze durch die selbstschließende Membran eingeführt und mit dieser Außenluft über den sterilen Filter angesaugt, so steht die hernach in einem zur Spitze gehörenden Reservoir vorhandene sterile Luft für eine Applikation in den mindestens einen Zugang zur Verfügung, somit kann vergleichsweise einfach sterile Luft für eine Spülung bereitgestellt werden, ohne dass sterile Luft vorgehalten werden muss. Alternativ kann auch ein Luftanschluss eines gängigen Luftanschlusssystems, in dem sterile Luft vorhanden ist, vorgesehen sein und diese über die Spitze in das Reservoir gezogen werden.

In alternativen Ausführungsformen ist der Luftanschluss fluidisch mit dem Verbindungsstück verbunden, so dass der mindestens eine Zugang, der Luftanschluss und die mindestens eine Leitung miteinander über das mindestens eine Verbindungsstück fluidisch verbunden sind. So können über in den Luftanschluss abgegebene Luft Zugang, Verbindungsstück und Leitung gespült werden. Auch hier kann der Luftanschluss sowohl als Anschluss eines Luftanschlusssystems ausgebildet sein als auch eine weitere selbstschließende Membran und einen sterilen Filter umfassen, insbesondere einen sterilen Filter mit einem Porendurchmesser kleiner oder gleich 0,22 µm. In letzterem Fall wird Luft beispielsweise über eine weitere Spitze durch die Membran appliziert und vom sterilen Filter gefiltert in das Verbindungsstück appliziert.

In einer Ausführungsform umfasst der Multi-Connector-Port mehrere am Verbindungsstück angeordnete Zugänge. Das Vorsehen mehrerer Zugänge erlaubt beispielsweise die gleichzeitige Zugabe verschiedener Medien aber auch die gleichzeitige Entnahme mehrerer Proben.

In einer weiteren Ausführungsform weist der Multi-Connector-Port an jedem Verbindungsstück genau einen Zugang und genau eine Leitung auf. In dieser Ausführungsform werden die über die Zugänge zugegebenen oder entnommene Medien vom jeweiligen Zugang bis zum verbundenen Gefäß, beispielsweise dem Bioreaktor, vollständig getrennt geführt. Damit können beispielsweise Vermischungen vor Eintritt in den Bioreaktor vermieden werden. Es ist insbesondere bevorzugt, wenn der Multi-Connector-Port genau zwei Verbindungsstücke aufweist, an denen jeweils ein Zugang und eine Leitung angeordnet sind. Weiterhin ist es bevorzugt, wenn ein Zugang für eine Zugabe von Medien und ein Zugang für eine Entnahme von Medien ausgebildet ist. In dieser Ausführungsform können Proben entnommen werden, ohne dass die Gefahr von Verfälschungen durch zuvor zugegebenen Medien besteht.

Der Multi-Connector-Port weist in einer Ausführungsform eine Vielzahl von Zugängen und Verbindungsstücken auf. Auch in dieser Ausführungsform ist nur ein Luftanschluss notwendig, dieser kann zur Spülung für alle Zugänge verwendet werden. Hier ist es insbesondere vorteilhaft, wenn der Luftanschluss derart ausgebildet ist, dass durch ihn sterile Luft in eine Spitze angesaugt werden kann. Diese Ausführungsform ist insbesondere vorteilhaft, da sie mit einer Vielzahl von Bioreaktoren gleichzeitig eingesetzt werden kann.

In weiteren Ausführungsformen sind der jeweilige Zugang und die jeweilige Leitung sowie der Luftanschluss über das jeweilige Verbindungsstück fluidisch verbunden. Damit ist eine Spülung über einen Luftanschluss in beiden Zugängen möglich, die Führung der Medien zwischen Gefäß und Zugang erfolgt aber getrennt voneinander, so dass Vermischungen oder Kontaminationen vermieden werden.

In einer Ausführungsform weist die mindestens eine Leitung des Multi-Connector-Ports an ihrem dem Verbindungsstück abgewandten Ende einen gas- und flüssigkeitsdichten Anschluss auf. Über diesen Anschluss kann der Multi-Connector-Port einfach und sicher beispielsweise an die Kopfplatte eines Bioreaktors angeschlossen werden. Hier sind insbesondere standardisierte Anschlüsse, die mit möglichst vielen Behältern oder Kopfplatten kompatibel sind von Vorteil. Beispielsweise kann es sich um einen Luer-Lock-Anschluss handeln oder Schraubanschlüsse. In einer sehr einfachen Ausgestaltung ist die mindestens eine Leitung als einfacher Schlauch ausgeführt, der beispielsweise in einen Anschluss einer Kopfplatte eines Bioreaktors eingesteckt werden kann.

Die selbstschließende Membran des Multi-Connector-Ports ist in einer Ausführungsform ebenfalls als durchstechbares Septum ausgebildet. Es ist insbesondere bevorzugt, wenn das durchstechbare Septum für Gase und Flüssigkeiten bis zu einem Druck von 0,5 bar undurchlässig ist, auch wenn das Septum von einer Hohlnadel oder einer Pipettenspitze, insbesondere von einer Hohlnadel oder Pipettenspitze mit einem Durchmesser unter 1,5 mm, penetriert wird und auch nach mehrfacher Penetration durch eine derartige Hohlnadel oder Pipettenspitze. Das durchstechbare Septum weist in einer Ausführungsform einen Schlitz, insbesondere einen Kreuzschlitz für die Penetration auf. Das Septum kann beispielsweise aus Silikon, Naturkautschuk, PTFE, TPE oder anderen Elastomeren gefertigt sein, derartige Septen sind aus dem Stand der Technik bereits bekannt. Der Zugang kann in einer alternativen Ausführungsform aber auch in als nadelfreies Membranventil umfassend die selbstschließende Membran ausgebildet sein, derartige Ventile sind beispielsweise aus US 5,368,801 A, US 7,9497,032 B2 oder WO 2013/158756 bekannt.

Gemäß einem vierten Aspekt betrifft die Erfindung ein Verfahren zum Betrieb eines Sterilschutz-Aufsatzes gemäß dem ersten Aspekt der Erfindung umfassend die Schritte
- Einbringen eines Endes einer Spitze einer Liquid-Handling-Einrichtung in den inneren Hohlraum der Schutzglocke des Sterilschutz-Aufsatzes, wobei die Spitze durch die Aufnahme der Schutzglocke geführt wird;
- Umspülen des Endes der Spitze mit steriler Luft aus der Zuleitung für sterile Luft.

In einer Ausführungsform des Verfahrens umfasst das Einbringen des Endes der Spitze ein Penetrieren der Aufnahme der Schutzglocke. Dies ist insbesondere vorteilhaft, wenn die Aufnahme eine selbstschließende Membran oder eine Dichtung umfasst.

Vorteilhaft wird die Spitze vor dem Einbringen des Endes in den inneren Hohlraum gereinigt, insbesondere durch Abwischen oder Spülen mit einem Reinigungsmittel umfassend z.B. 70% Isopropanol.

In einer Ausführungsform des Verfahrens wird der gesamte Sterilschutz-Aufsatz oder auch nur die Schutzglocke vor dem Einbringen der Spitze sterilisiert. Dies kann entweder unmittelbar vordem Einsatz erfolgen oder durch die Nutzung einer steril-verpackten austauschbaren Schutzglocke oder steril-verpackten austauschbaren Sterilschutz-Aufsatzes.

Gemäß einem fünften Aspekt betrifft die Erfindung ein Verfahren zum Befüllen oder Entnehmen mit einer Sterilschutzeinheit gemäß dem zweiten Aspekt der Erfindung umfassend die Schritte,
- Einbringen eines Endes der Spitze der Liquid-Handling-Einrichtung in den inneren Hohlraum der Schutzglocke des Sterilschutz-Aufsatzes, wobei die Spitze in der Aufnahme der Schutzglocke angeordnet wird;
- Umspülen des Endes der Spitze mit steriler Luft aus der Zuleitung für sterile Luft;
- Reinigen einer Außenfläche eines Anschlusses oder Gefäßes, insbesondere durch Abwischen oder Spülen mit einem Reinigungsmittel umfassend insbesondere Isopropanol oder Ethanol,
- Penetrieren des Anschlusses oder Gefäßes mit der Spitze,
- Zugeben eines Mediums in den Anschluss oder das Gefäß oder Absaugen eines Mediums aus dem Anschluss oder Gefäß über die Spitze,
- Entfernen der Spitze aus dem Anschluss oder Gefäß.

In einer Ausführungsform umfasst das Verfahren weiter den Schritt
- beim Penetrieren des Anschlusses oder Gefäßes durch die Spitze, Stoppen des Umspülens sowie optional
- Wiederaufnehmen des Umspülens, sobald die Spitze aus dem Gefäß oder Anschluss entfernt wird.

Das Stoppen des Umspülens kann insbesondere vorteilhaft sein, um zu vermeiden, dass noch auf der Außenfläche des Anschlusses oder Gefäßes befindliches Reinigungsmittel durch den Luftstrom im Raum verteilt wird.

In einer Ausführungsform des Verfahrens wird beim Penetrieren des Anschlusses oder Gefäßes mit der Spitze diese Schutzglocke auf einen vorgegebenen Abstand zum Anschluss oder Gefäß bewegt, so dass ein Aufsetzen der Schutzglocke und damit eine mögliche Kontamination der Schutzglocke vermieden wird. In alternativen Ausführungsformen wird die Schutzglocke aufgesetzt, insbesondere gasdicht aufgesetzt.

Die Spitze wird in einer weiteren Ausführungsform nach dem Ende des Penetrierens des Gefäßes oder Anschlusses in Relation zur Schutzglocke nur soweit zurückgezogen, dass das Ende der Spitze innerhalb des Hohlraums der Schutzglocke verbleibt.

In einer Ausführungsform des Verfahrens umfasst das Einbringen des Endes der Spitze ein Penetrieren der Aufnahme der Schutzglocke. Dies ist insbesondere vorteilhaft, wenn die Aufnahme eine selbstschließende Membran oder eine Dichtung umfasst.

Vorteilhaft wird die Spitze vor dem Einbringen des Endes in den inneren Hohlraum gereinigt, insbesondere durch Abwischen oder Spülen mit einem Reinigungsmittel umfassend z.B. 70% Isopropanol.

In einer Ausführungsform des Verfahrens wird der gesamte Sterilschutz-Aufsatz oder auch nur die Schutzglocke vor dem Einbringen der Spitze sterilisiert. Dies kann entweder unmittelbar vor dem Einsatz erfolgen oder durch die Nutzung einer steril-verpackten austauschbaren Schutzglocke oder steril-verpackten austauschbaren Sterilschutz-Aufsatzes.

Gemäß einem sechsten Aspekt betrifft die Erfindung ein Verfahren zum Befüllen oder Entnehmen mit einem Liquid-Handling-System gemäß dem dritten Aspekt der Erfindung mit mindestens einem Multi-Connector-Port, wobei der Multi-Connector-Port einen Luftanschluss für sterile Luft sowie mindestens einen an einem Verbindungsstück angeordneten Zugang, der eine selbstschließende Membran umfasst und mindestens eine am Verbindungstück angeordnete Leitung umfasst, umfassend die Schritte
- Einbringen eines Endes der Spitze der Liquid-Handling-Einrichtung in den inneren Hohlraum der Schutzglocke des Sterilschutz-Aufsatzes;
- Umspülen des Endes der Spitze mit steriler Luft aus der Zuleitung für sterile Luft;
- Verbinden der mindestens einen Leitung des Multi-Connector-Ports mit einem Gefäß, insbesondere einem Bioreaktor oder einer Vorlageflasche,
- Reinigen einer Außenfläche des mindestens einen Zugangs und/oder einer Außenfläche des Luftanschlusses, insbesondere durch Abwischen oder Spülen mit einem Reinigungsmittel umfassend insbesondere Isopropanol oder Ethanol,
- Penetrieren der selbstschließenden Membran des mindestens einen Zugangs mit einer Spitze, insbesondere in Form einer Hohlnadel, einer Pipettenspitze oder einem männlichen Verbindungsstück,
- Zugeben eines Mediums in das Gefäß oder Absaugen eines Mediums aus dem Gefäß über die Spitze,
- Entfernen der Spitze.

Bevorzugt umfasst das Verfahren gemäß dem sechsten Aspekt weiterhin den Schritt Applikation von steriler Luft in den Zugang. Bevorzugt umfasst die Applikation von steriler Luft in den Zugang dabei folgende Schritte:
- Penetrieren der weiteren selbstschließenden Membran des Luftanschlusses mit einer Spitze
- Ansaugen von Umgebungsluft durch den sterilen Filter über die Spitze in ein mit der Spitze verbundenes Reservoir
- Entfernen der Spitze aus der weiteren selbstschließenden Membran,
- Penetrieren der selbstschließenden Membran des mindestens einen Zugangs des Multi-Connector-Ports mit der Spitze
- Applizieren von Luft aus dem Reservoir in den Zugang.

Über dieses Verfahren wird es im Zusammenspiel mit den weiteren Komponenten des Liquid-Handling-Systems einfach ermöglicht, sterile Luft für die Spülung zu verwenden und damit Totvolumina zu vermeiden, ohne dass sterile Luft vorgehalten werden muss. Damit kann das Arbeiten noch flexibler und unabhängiger von weiterer Infrastruktur erfolgen.

Alternativ erfolgt die Applikation von steriler Luft über den Luftanschluss, indem sterile Luft über den Luftanschluss in das mit dem Luftanschluss fluidisch verbundene mindestens eine Verbindungstück appliziert wird, wobei entweder sterile Luft von außen über den Luftanschluss zugeführt wird oder Luft über eine weitere selbstschließende Membran und einen sterilen Filter in das mindestens eine Verbindungsstück und die daran angeordnete mindestens eine Leitung sowie den mindestens einen Zugang appliziert wird.

In einer Ausführungsform des Verfahrens umfasst das Einbringen des Endes der Spitze ein Penetrieren der Aufnahme der Schutzglocke. Dies ist insbesondere vorteilhaft, wenn die Aufnahme eine selbstschließende Membran oder eine Dichtung umfasst.

Vorteilhaft wird die Spitze vor dem Einbringen des Endes in den inneren Hohlraum gereinigt, insbesondere durch Abwischen oder Spülen mit einem Reinigungsmittel umfassend insbesondere Isopropanol oder Ethanol z.B. 70% Isopropanol.

In einer Ausführungsform des Verfahrens wird der gesamte Sterilschutz-Aufsatz oder auch nur die Schutzglocke vor dem Einbringen der Spitze sterilisiert. Dies kann entweder unmittelbar vor dem Einsatz erfolgen oder durch die Nutzung einer steril-verpackten austauschbaren Schutzglocke oder steril-verpackten austauschbaren Sterilschutz-Aufsatzes.

In einer bevorzugten Ausführungsform erfolgt nach der Applikation steriler Luft eine abschließende Reinigung der Außenfläche des mindestens einen Zugangs und/oder der Außenfläche des Luftanschlusses. Die Reinigung erfolgt dabei bevorzugt mit Isopropanol oder ähnlichen Reinigungsflüssigkeiten und/oder einem Desinfektionsmittel. Der Reinigungsschritt vereinfacht es, mit dem Multi-Connector-Port auch außerhalb steriler Umgebungen zu arbeiten und dennoch das Befüllen und Entnehmen unter sterilen Bedingungen zu gewährleisten. Dabei ist es bevorzugt, wenn der der Multi-Connector-Port mindestens eine Kappe umfasst und diese vor dem Reinigen abgenommen wird und/oder nach der abschließenden Reinigung aufgesetzt wird.

In einer weiteren Ausführungsform erfolgen weiter die Schritte
- beim Penetrieren der selbstschließenden Membran des mindestens einen Zugangs durch die Spitze, Stoppen des Umspülens sowie optional
- Wiederaufnehmen des Umspülens, sobald die Spitze aus dem Gefäß oder Anschluss entfernt wird.

Vorteilhaft wird die Spitze nach dem Ende des Penetrierens der selbstschließenden Membran des mindestens einen Zugangs in Relation zur Schutzglocke nur soweit zurückgezogen wird, dass das Ende der Spitze innerhalb des Hohlraums der Schutzglocke verbleibt.

Die Sterilschutzeinheit gemäß dem zweiten Aspekt der Erfindung, das Liquid-Handling-System gemäß dem dritten Aspekt, sowie die Verfahren nach dem vierten, fünften und sechsten Aspekt teilen die Vorteile und Ausführungsformen des Sterilschutz-Aufsatzes gemäß dem ersten Aspekt.

Nachfolgend werden weitere Ausführungsformen beispielhaft anhand der beiliegenden Figuren erläutert. Es zeigen:
Fig. 1: eine Ausführungsform einer Sterilschutzeinheit gemäß dem zweiten Aspekt der Erfindung;
Fig. 2a und 2b: Detailansichten der Ausführungsform der Sterilschutzeinheit aus Fig. 1;
Fig. 3: eine Ausführungsform eines Liquid-Handling-Systems gemäß dem dritten Aspekt der Erfindung mit einem Multi-Connector-Port.

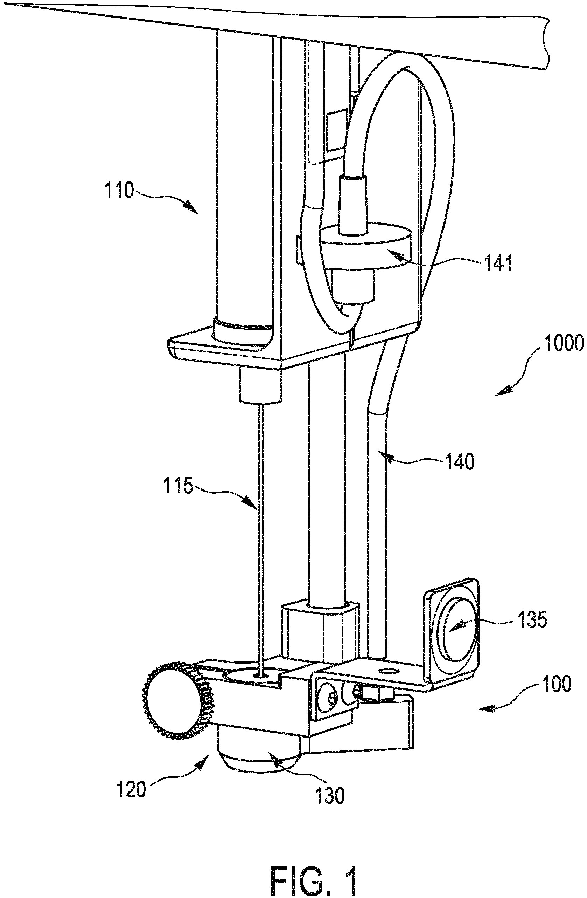

Fig. 1 zeigt eine Ausführungsform einer Sterilschutzeinheit 1000 gemäß dem zweiten Aspekt der Erfindung mit einem Sterilschutz-Aufsatz 100 und einer Spitze 115 einer Liquid-Handling-Einrichtung 110. Die Spitze ist hier eine Hohlnadel. In der gezeigten Ausführungsform umfasst der Sterilschutz-Aufsatz 100 eine Halterung 120 zur Befestigung des Sterilschutz-Aufsatzes 100 an der Liquid-Handling-Einrichtung 110. Hierin ist eine Schutzglocke 130 angeordnet. In der gezeigten Ausführungsform ist die Schutzglocke 130 über eine Klemmvorrichtung mit einer Stellschraube in der Halterung angeordnet und befestigt. Die Schutzglocke 130 weist einen inneren Hohlraum auf. Mit diesem ist eine Zuleitung 140 für sterile Luft verbunden. Über die Zuleitung wird der innere Hohlraum der Schutzglocke mit steriler Luft geflutet und so die Spitze gegen eine nicht sterile Umgebung gekapselt. Die Schutzglocke 130 weist an einem unteren Ende eine Öffnung auf, und an einer dem unteren Ende gegenüberliegenden oberen Seite eine Aufnahme für die Spitze 115. Durch die untere Öffnung kann sterile Luft entweichen, die gleichzeitig über die Zuleitung 140 nachgeführt wird. In der gezeigten Ausführungsform ist die Schutzglocke aus Silikon gefertigt und damit insbesondere an ihrem unteren Ende so elastisch, dass sie beim Aufsetzen auf ein Gefäß oder einen Anschluss mit diesem dicht abschließt. In weiteren Ausführungsformen, bei denen das untere Ende der Schutzglocke nicht für einen dichten Abschluss ausgebildet ist, wird die Spitze permanent von steriler Luft umspült, auch während des Penetrierens der Spitze in einen Anschluss oder ein Gefäß. Hierüber wird im Betrieb jederzeit sichergestellt, dass die Spitze gegen die Umgebung gekapselt bleibt. Ist das untere Ende elastisch ausgebildet und in der Lage gasdicht mit dem Anschluss oder Gefäß abzuschließen, kann die Zufuhr an steriler Luft während des Penetrierens auch gestoppt werden und erst beim Herausfahren der Spitze wieder aktiviert werden.

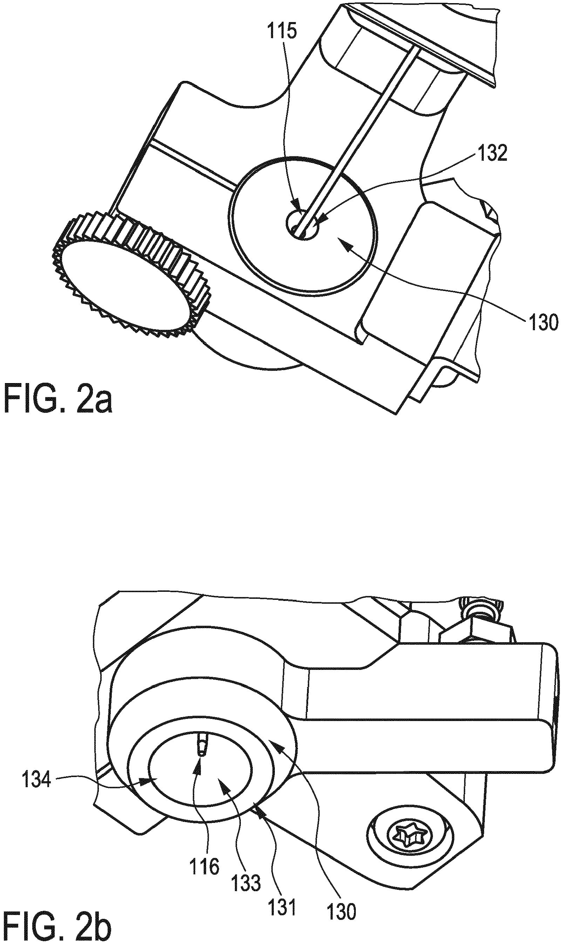

In den Fig. 2a und 2b sind Detailansichten der Sterilschutzeinheit dargestellt. Fig. 2a zeigt die Aufnahme 132 der Schutzglocke 130, in der sich die Spitze 115 befindet. In der gezeigten Ausführungsform ist die Aufnahme eine Öffnung, durch die die Spitze hindurch geführt ist. Durch die Spülung des inneren Hohlraumes 133 der Schutzglocke 130 mit steriler Luft wird sichergestellt, dass die Spitze innerhalb des inneren Hohlraumes nicht kontaminiert wird. In Fig. 2b ist das untere offene Ende 116 der Spitze 115 im inneren Hohlraum 133 der Schutzglocke 130 dargestellt. Die Öffnung 134 am unteren Ende der Schutzglocke 130 ist hier kreisförmig ausgebildet. Die Spitze 115 ist relativ zu Schutzglocke 130 beweglich angeordnet und zwar derart, das ihr unteres offenes Ende 116 beim Penetrieren eines Anschlusses oder Gefäßes unterhalb der Schutzglocke angeordnet ist. So kann das untere offene Ende 116 der Spitze 115 in einen Anschluss oder ein Gefäß eingeführt werden während der im Hohlraum befindliche Teil der Spitze weiterhin von steriler Luft aus der Zuleitung gegen Kontaminationen aus der Umgebungsluft geschützt ist. Damit ist das sterile Applizieren und Entnehmen aus einem Gefäß oder Anschluss möglich ohne dass in steriler Umgebung gearbeitet werden muss.

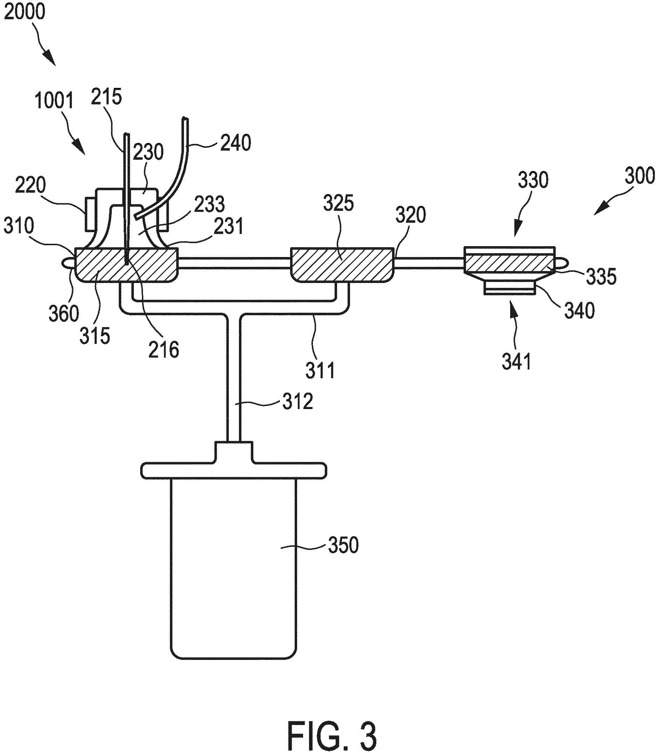

Fig. 3 zeigt eine Ausführungsform eines Liquid-Handling-Systems 2000 gemäß dem dritten Aspekt der Erfindung mit einer Sterilschutzeinheit 1001 umfassend eine Spitze 215 einer Liquid-Handling-Einrichtung mit einem unteren offenen Ende 216 sowie einem Sterilschutz-Aufsatz. Der Sterilschutz-Aufsatz umfasst eine Halterung 220, mit der der Sterilschutz-Aufsatz an der Liquid-Handling-Einrichtung befestigt ist, sowie eine Schutzglocke 230. Die Schutzglocke 230 sitzt im gezeigten Zustand auf einem Zugang eines Multi-Connector-Ports 300 des Liquid-Handling-Systems 2000 auf. Ihr unteres Ende 231 ist elastisch ausgebildet und wird beim Aufsetzen auf den Zugang leicht nach außen gebogen, so dass sich ein gasdichter Abschluss ergibt. Über eine Zuleitung 340 für sterile Luft wird der innere Hohlraum 233 der Schutzglocke 230 mit steriler Luft gespült und so die Spitze 215 gegen die Umgebung gekapselt. Zusammen mit dem Multi-Connector-Port 300 ergibt sich ein flexibles Liquid-Handling-System, mit dem auch in nicht-steriler Umgebung steril gearbeitet werden kann.

Die hier gezeigte Ausführungsform des Multi-Connector-Ports 300 weist ein Verbindungsstück 311 auf, an dem zwei Zugänge 310, 320 mit jeweils einer selbstschließenden Membran 315, 325 angeordnet sind. Der Multi-Connector-Port 300 weist auch einen Luftanschluss für sterile Luft 330 auf, der eine weitere selbstschließende Membran 335 sowie einen sterilen Filter 340 aufweist. Des Weiteren weist der Multi-Connector-Port 300 eine Fixiervorrichtung 360 für eine Integration in ein Handlingsystem auf. Der Luftanschluss 330 ist hier nicht fluidisch mit dem Verbindungsstück 311 verbunden. In der gezeigten Ausführungsform hat der sterile Filter 340 auf seiner der weiteren selbstschließenden Membran abgewandten Seite 341 Kontakt zur Außenluft.

Nachfolgend erläutern wir die Arbeitsweise des gezeigten Ausführungsbeispiels eines Liquid-Handling-Systems 2000. Wie gezeigt wird das untere offene Ende 216 der Spitze 215 in den Zugang 310 eingebracht und penetriert dabei die selbstschließende Membran 315, welche sich dicht um die Spitze schließt beim Penetrieren. Das untere offene Ende 216 wird dann vollständig durch die selbstschließende Membran 315 geführt. Nunmehr wird beispielsweise ein Medium in den Zugang appliziert. Dabei kann in der gezeigten Ausführungsform die Spülung mit steriler Luft im inneren Hohlraum unterbrochen sein oder auch dauerhaft durchgeführt werden. Nach der Applikation wird die Spitze 215 zurückgezogen. Ihr unteres offenes Ende 216 befindet sich dann wieder im gespülten inneren Hohlraum 233. Nunmehr kann die Spitze zum Luftanschluss 330 verfahren werden, wobei sie auch während des Verfahrens durch die sterile Luft im inneren Hohlraum gegen die Umgebung gekapselt bleibt.

Wird durch die selbstschließende Membran 330 die Spitze 215 von oben eingeführt, so kann über diese Luft durch den sterilen Filter 340 angesaugt und in ein mit der Spitze verbundenes Reservoir gegeben werden. Auch hierbei wird die Spitze 215 im inneren Hohlraum durch die sterile Luft und die Schutzglocke gegen die Umgebungsluft gekapselt. Hernach kann die Spitze wieder in einen der Zugänge 310, 320, hier Zugang 310 eingebracht werden und damit Luft in Verbindungsstück 311 und Leitung 312 appliziert werden, so dass in Zugang, Verbindungsstück oder Leitung befindliches Medium aus dem vorherigen Befüll- oder Entnahmevorgang in das hier verbundene Reaktionsgefäß 350 gedrückt wird.

## Patentansprüche

1. Sterilschutz-Aufsatz (100) für eine Spitze (115) einer Liquid-Handling-Einrichtung (110) umfassend eine Halterung (120) zur Befestigung des Sterilschutz-Aufsatzes (100) an der Liquid-Handling-Einrichtung (110), eine in der Halterung angeordnete Schutzglocke (130) mit einem inneren Hohlraum (133) sowie eine mit dem Hohlraum verbundene Zuleitung (140) für sterile Luft, bei dem die Schutzglocke (130) an einem unteren Ende (131) eine Öffnung (134) aufweist und an einer dem unteren Ende gegenüberliegenden oberen Seite eine Aufnahme (132) für die Spitze (115) aufweist.

2. Sterilschutz-Aufsatz nach Anspruch 1, bei dem die Aufnahme eine Öffnung ist, durch die die Spitze geführt werden kann oder bei dem die Aufnahme ausgebildet ist, die Spitze gas- und flüssigkeitsdicht zu umschließen.

3. Sterilschutz-Aufsatz nach einem der vorstehenden Ansprüche, bei dem die Schutzglocke ganz oder teilweise aus Polyetheretherketon gefertigt ist.

4. Sterilschutz-Aufsatz nach einem der vorstehenden Ansprüche, bei dem die Halterung längenverstellbar, insbesondere in Teleskopform, ausgeführt ist.

5. Sterilschutz-Aufsatz nach einem der vorstehenden Ansprüche, bei dem die Zuleitung für sterile Luft einen sterilen Filter, insbesondere einen sterilen Filter mit einem Porendurchmesser kleiner oder gleich 0,22 µm umfasst.

6. Sterilschutzeinheit (1000) umfassend einen Sterilschutz-Aufsatz nach einem der vorstehenden Ansprüche sowie eine in der Aufnahme der Schutzglocke angeordnete Spitze einer Liquid-Handling-Einrichtung.

7. Sterilschutzeinheit nach Anspruch 6, bei der die Halterung derart ausgebildet und angeordnet ist, dass ein unteres offenes Ende (116) der Spitze im Betrieb dauerhaft innerhalb des Hohlraums der Schutzglocke angeordnet ist und - nur bei Penetration der Spitze in ein Gefäß oder einen Anschluss- unterhalb des Hohlraums der Schutzglocke angeordnet ist.

8. Sterilschutzeinheit nach einem Ansprüche 6 oder 7, bei der die Spitze im Betrieb relativ zur Schutzglocke beweglich angeordnet ist.

9. Sterilschutzeinheit nach einem Ansprüche 6 oder 7, bei der Spitze starr zur Halterung angeordnet ist und das untere Ende der Schutzglocke elastisch ausgebildet ist.

10. Liquid-Handling-System (2000) umfassend mindestens eine Liquid-Handling-Einrichtung mit mindestens einer Sterilschutzeinheit nach einem der Ansprüche 6 bis 9.

11. Liquid-Handling-System nach Anspruch 10 weiter umfassend mindestens einen Multi-Connector-Port (300), wobei der Multi-Connector-Port einen Luftanschluss (330) für sterile Luft sowie mindestens einen an einem Verbindungsstück (311) angeordneten Zugang (310, 320), der eine selbstschließende Membran umfasst und mindestens eine am Verbindungstück angeordnete Leitung (312) umfasst.

12. Verfahren zum Betrieb eines Sterilschutz-Aufsatzes nach einem der Ansprüche 1 bis 5 umfassend die Schritte
- Einbringen eines Endes einer Spitze einer Liquid-Handling-Einrichtung in den inneren Hohlraum der Schutzglocke des Sterilschutz-Aufsatzes, wobei die Spitze durch die Aufnahme der Schutzglocke geführt wird;
- Umspülen des Endes der Spitze mit steriler Luft aus der Zuleitung für sterile Luft.

13. Verfahren zum Befüllen oder Entnehmen mit einer Sterilschutzeinheit nach einem der Ansprüche 6 bis 9 umfassend die Schritte,
- Einbringen eines Endes der Spitze der Liquid-Handling-Einrichtung in den inneren Hohlraum der Schutzglocke des Sterilschutz-Aufsatzes, wobei die Spitze in der Aufnahme der Schutzglocke angeordnet wird;
- Umspülen des Endes der Spitze mit steriler Luft aus der Zuleitung für sterile Luft;
- Reinigen einer Außenfläche eines Anschlusses oder Gefäßes, insbesondere durch Abwischen oder Spülen mit einem Reinigungsmittel umfassend insbesondere Isopropanol oder Ethanol,
- Penetrieren des Anschlusses oder Gefäßes mit der Spitze,
- Zugeben eines Mediums in den Anschluss oder das Gefäß oder Absaugen eines Mediums aus dem Anschluss oder Gefäß über die Spitze,
- Entfernen der Spitze aus dem Anschluss oder Gefäß.

14. Verfahren zum Befüllen oder Entnehmen mit einer Sterilschutzeinheit nach Anspruch 13, weiter umfassend den Schritt
- beim Penetrieren des Anschlusses oder Gefäßes durch die Spitze, Stoppen des Umspülens sowie optional
- Wiederaufnehmen des Umspülens, sobald die Spitze aus dem Gefäß oder Anschluss entfernt wird.

15. Verfahren zum Befüllen oder Entnehmen mit einem Liquid-Handling-System nach Anspruch 11 umfassend die Schritte,
- Einbringen eines Endes der Spitze der Liquid-Handling-Einrichtung in den inneren Hohlraum der Schutzglocke des Sterilschutz-Aufsatzes;
- Umspülen des Endes der Spitze mit steriler Luft aus der Zuleitung für sterile Luft;
- Verbinden der mindestens einen Leitung des Multi-Connector-Ports mit einem Gefäß, insbesondere einem Bioreaktor oder einer Vorlageflasche,
- Reinigen einer Außenfläche des mindestens einen Zugangs und/oder einer Außenfläche des Luftanschlusses, insbesondere durch Abwischen oder Spülen mit einem Reinigungsmittel umfassend insbesondere Isopropanol oder Ethanol,
- Penetrieren der selbstschließenden Membran des mindestens einen Zugangs mit einer Spitze, insbesondere in Form einer Hohlnadel, einer Pipettenspitze oder einem männlichen Verbindungsstück,
- Zugeben eines Mediums in das Gefäß oder Absaugen eines Mediums aus dem Gefäß über die Spitze,
- Entfernen der Spitze,
- Applikation von steriler Luft.
